# EUROPEAN PATENT SPECIFICATION

(11) **EP 2 842 518 B1**
(45) Date of publication and mention of the grant of the patent: **05.12.2018**
(21) Application number: 13782004.9
(22) Date of filing: 28.03.2013
(51) Int. Cl.: A61F 2/30, C08F 2/48, A61F 2/34, B05D 3/06, B05D 7/22

(54) **FILM-PRODUCING DEVICE AND METHOD FOR PRODUCING ARTIFICIAL JOINT COMPOSITE**
FILMERZEUGENDE VORRICHTUNG UND VERFAHREN ZUR HERSTELLUNG EINES KÜNSTLICHEN GELENKVERBUNDS
DISPOSITIF DE FABRICATION D'UNE PELLICULE ET PROCÉDÉ DE PRODUCTION D'UN COMPOSITE POUR PROTHÈSE ARTICULAIRE

(30) Priority: 27.04.2012 JP 2012103082
(43) Date of publication of application: 04.03.2015
(73) Proprietor: Kyocera Corporation, Kyoto City (JP)
(72) Inventor: NAMBA,Ryota, Osaka-shi Osaka 532-0003 (JP)
(74) Representative: Isarpatent
(86) International application number: PCT/JP2013/059196
(87) International publication number: WO 2013/161499

(56) References cited:
- EP-A1- 2 856 978
- JP-A- 2000 217 844
- JP-A- 2008 148 850
- JP-A- 2010 056 192
- JP-A- 2011 197 544
- US-A1- 2005 154 075

## Description

### Technical Field

The present invention relates to a film-producing device used in production of an artificial joint composite, and a method for producing an artificial joint composite.

### Background Art

Attempts have been made to form a sliding surface of an artificial joint composite with a coating layer, for the purpose of improving the abrasion resistance of an artificial joint. As a method for forming such a coating layer, a method is known in which a macromolecular layer is formed by means of photograft polymerization, on a surface of a base material that serves as a main body of an artificial joint composite (e.g., see JP 2011-197544A).

Furthermore, US 2005/015475 A1 describes a method for UV printing and curing of CDs, DVDs, Golf Balls and other products. The method is performed by emitting substantially uniform intermittent blasts or pulses of UV light along at least one UV light path, wherein UV curable products are positioned within the at least one UV light path.

### Disclosure of the Invention

### Problem to be Solved by the Invention

JP 2011-197544A discloses a configuration in which a photograft polymerization reaction is caused by irradiating, with light, a solution that contains macromolecular monomers while a curved surface of the base material is in contact with the solution. A macromolecular layer is formed on the curved surface of the base material by means of photograft polymerization. JP 2011-197544A discloses a configuration in which a UV lamp (high-pressure mercury lamp) is used for light irradiation.

However, in the case of using a high-pressure mercury lamp, the size of the high-pressure mercury lamp itself is large relative to the size of the base material of the artificial joint composite, each side of which is several centimeters long. For this reason, the size of the film-producing device increases. Furthermore, in the case of using a high-pressure mercury lamp, a lamp cooling device such as a chiller for cooling the high-pressure mercury lamp is necessary. For this reason, the size of the film-producing device further increases.

With a high-pressure mercury lamp, generally, the ultraviolet intensity cannot be finely adjusted by changing the voltage or current. Therefore, the intensity of the light with which the solution is irradiated needs to be adjusted by finely adjusting the distance between the large high-pressure mercury lamp and the surface of the base material. Accordingly, it is difficult to accurately control the ultraviolet irradiation intensity.

With a configuration in which photopolymerization is carried out using a high-pressure mercury lamp, it is difficult to form a macromolecular layer having a uniform thickness on the surface of the base material. This is because the light intensity at a position close to the center of the lamp is significantly different from the light intensity at a position far from the center of the lamp, in a plane facing the high-pressure mercury lamp. Moreover, since the high-pressure mercury lamp is a linear light source, it is difficult to uniformly irradiate, with ultraviolet light, the surface of the base material in portions other than portions close to the center of the lamp.

A high-pressure mercury lamp generates ultraviolet-B, which is considered unnecessary for photograft polymerization. As a result of irradiation with ultraviolet-B, deterioration of equipment that receives the ultraviolet light from the high-pressure mercury lamp, such as a holding member for holding the base material, will be caused. Furthermore, exposing workers who produce artificial joint composites to ultraviolet light that is unnecessary for photograft polymerization is not advisable in terms of the health management of the workers.

The light-emitting portion of a high-pressure mercury lamp is made of glass. Moreover, a cooling pipe made of glass or the like, for example, is arranged around the high-pressure mercury lamp. For this reason, the high-pressure mercury lamp is likely to malfunction due to breakage or the like. JP 2011-197544A also mentions using an LED (Light Emitting Diode), apart from a high-pressure mercury lamp, as a configuration for light irradiation. However, JP 2011-197544A does not disclose any specific configuration for irradiating the base material with the light of the LED.

In view of the foregoing situation, an object of the present invention is, with regard to a configuration for forming a macromolecular layer on a surface of a base material of an artificial joint composite, to achieve a size reduction, to accurately control ultraviolet irradiation intensity, to form a macromolecular layer having a uniform thickness, to suppress the influence of ultraviolet light on equipment and workers, and to make the occurrence of a malfunction unlikely.

### Means for Solving the Problem

The invention provides a film-producing device with the features of claim 1 and a method for producing an artificial joint composite with the features of claim 4.

According to this aspect of the invention, ultraviolet light is emitted by the ultraviolet LED elements. Each ultraviolet LED element is formed to have a sufficiently small shape relative to the artificial joint composite. Accordingly, it is not necessary to use a high-pressure mercury lamp or the like having a significantly larger shape than the shape of the base material of the artificial joint composite, in order to produce the artificial joint composite. Furthermore, since the shape of each ultraviolet LED element is small, even if a plurality of ultraviolet LED elements are arranged, the entire space occupied by the ultraviolet LED elements is small. Moreover, the amount of the heat generated by the ultraviolet LED elements is significantly smaller than that of a high-pressure mercury lamp, and it is not necessary to prepare a large-scale cooling device such as a chiller. As a result, a reduction in the size of the film-producing device can be achieved through a reduction in the size of the configuration for emitting ultraviolet.

With ultraviolet LED elements, the brightness of the ultraviolet LED elements can be easily finely adjusted by means such as adjusting the current supplied to the ultraviolet LED elements. For this reason, the illuminance of the ultraviolet light in the solution can be easily accurately adjusted. As a result, a macromolecular layer having a uniform thickness can be easily formed.

Ultraviolet LED elements have a higher directivity of the emitted light than that of a high-pressure mercury lamp, and can emit ultraviolet light at a more uniform intensity. Moreover, the ultraviolet LED elements are arranged separately from each other. As a result, ultraviolet light of uniform intensity can be emitted over a wide area. The progress of photograft polymerization on the surface of the base material can thereby be made more uniform. Accordingly, a macromolecular layer having a uniform thickness can be formed on the surface of the base material.

The wavelength width of the ultraviolet light emitted by the ultraviolet LED elements is significantly narrower than the wavelength width of the ultraviolet light emitted by a high-pressure mercury lamp. For this reason, the ultraviolet LED elements can be configured not to emit ultraviolet-B, which is considered unnecessary for photograft polymerization. As a result, it is possible to suppress deterioration of equipment due to ultraviolet-B in the members of the film-producing device that are irradiated with light from the ultraviolet LED elements, such as the work holder. Moreover, it is possible to suppress the exposure of workers who produce the artificial joint composites to ultraviolet-B, which is also preferable in terms of the health management of workers.

Each ultraviolet LED element is, generally, installed on a strong rigid base plate, and is easier to handle than a high-pressure mercury lamp, which has a large fragile glass portion. Moreover, as mentioned above, a glass portion such as a chiller for cooling the ultraviolet LED elements is not necessary. As a result, with the ultraviolet LED elements, it is possible to make the occurrence of a malfunction due to breakage unlikely. Moreover, by using ultraviolet LED elements whose life span is several times (e.g., five times) longer than the life span of a high-pressure mercury lamp, fewer failures occur due to the life span of the ultraviolet LED elements.

Accordingly, according to the present invention, it is possible, in the film-producing device for an artificial joint composite, to achieve a size reduction, to accurately control ultraviolet irradiation intensity, to form a macromolecular layer having a uniform thickness, to suppress the influence of ultraviolet light on equipment and workers, and to make the occurrence of a malfunction unlikely.

According to this aspect of the invention, the plurality of ultraviolet LED elements can be collectively operated. The plurality of ultraviolet LED elements can thereby be collectively displaced with respect to the work and the work holder. Accordingly, it can be made easier for a worker to handle the ultraviolet LED elements.

A film-producing device according to a third aspect of the invention is the film-producing device in the first or second aspect of the invention, wherein the film-producing device is provided with an adjustment mechanism for adjusting illuminance of the ultraviolet light in the solution by differentiating an amount of electric power supplied to some of the ultraviolet LED elements from an amount of electric power supplied to the other of the ultraviolet LED elements.

According to this aspect of the invention, by enabling the illuminance of the ultraviolet light in the solution to be adjusted, the uniformity of the thickness of the macromolecular layer formed by means of photograft polymerization can be further increased. In particular, in the case where the surface of the work is uneven, adjustment of the illuminance of the ultraviolet light in accordance with the shape of the work is favorable for further increasing the uniformity of the thickness of the macromolecular layer formed on the work.

Moreover, the ultraviolet LED elements are configured using LEDs whose brightness can be easily adjusted by electric power adjustment. The illuminance of the ultraviolet light in the solution can thereby be easily adjusted. As a result, the illuminance of the ultraviolet light in the solution can be made more uniform.

In this case, preferably, a power supply device for supplying electric power to the ultraviolet LED elements is further provided, and the power supply device supplies different amounts of electric power to the plurality of ultraviolet LED elements.

With this configuration, the output of the ultraviolet LED elements can be adjusted.

Preferably, the film-producing device further includes a light shielding member that optically shields a space between the plurality of ultraviolet LED elements and the work holder from an outside space.

According to this aspect of the invention, light other than the light from the ultraviolet LED elements can be prevented from entering a space between the ultraviolet LED elements and the work holder. A non-uniform graft polymerization reaction in the solution due to unnecessary ultraviolet light from the outside can thereby be prevented. As a result, the thickness of the macromolecular layer can be made more uniform.

In the irradiation step, ultraviolet light is emitted by the ultraviolet LED elements. Each ultraviolet LED element is formed to have a sufficiently small shape relative to the artificial joint composite. Accordingly, it is not necessary to use a high-pressure mercury lamp or the like having a significantly larger shape than the shape of the base material of the artificial joint component, in
order to produce the artificial joint component. Furthermore, since the shape of each ultraviolet LED element is small, even if a plurality of ultraviolet LED elements are arranged, the entire space occupied by the ultraviolet LED elements is small. Moreover, the amount of the heat generated by the ultraviolet LED elements is significantly smaller than that of a high-pressure mercury lamp, and it is not necessary to prepare a large-scale cooling device such as a chiller. As a result, a reduction in the size of the configuration for emitting ultraviolet can be achieved.

With the ultraviolet LED elements, the brightness of the ultraviolet LED elements can be easily finely adjusted by means such as adjusting the current supplied to the ultraviolet LED elements. For this reason, the illuminance of the ultraviolet light in the solution can be easily accurately adjusted. As a result, a macromolecular layer having a uniform thickness can be easily formed.

The ultraviolet LED elements have a higher directivity of the emitted light than that of a high-pressure mercury lamp, and can emit ultraviolet light at a uniform intensity. Moreover, the ultraviolet LED elements are arranged separately from each other. As a result, ultraviolet light of uniform intensity can be emitted over a wide area. The progress of photograft polymerization on the surface of the base material can thereby be made more uniform. Accordingly, a macromolecular layer having a uniform thickness can be formed on the surface of the base material.

The wavelength width of the ultraviolet light emitted by the ultraviolet LED elements is significantly narrower than the wavelength width of the ultraviolet light emitted by a high-pressure mercury lamp. For this reason, the ultraviolet LED elements can be configured not to emit ultraviolet-B, which is considered unnecessary for photograft polymerization. As a result, it is possible to suppress deterioration of equipment due to ultraviolet-B, in the members of the film-producing device that are irradiated with the light from the ultraviolet LED elements. Moreover, it is possible to suppress the exposure of workers who produce the artificial joint components to ultraviolet-B, which is also preferable in terms of the health management of workers.

Each ultraviolet LED element is, in general, installed on a strong rigid base plate, and is easier to handle than the high-pressure mercury lamp, which has a large fragile glass portion. Moreover, as mentioned above, a glass portion such as a chiller for cooling the ultraviolet LED elements is not necessary. As a result, with the ultraviolet LED elements, it is possible to make the occurrence of a malfunction due to breakage unlikely. Moreover, with ultraviolet LED elements whose life span is several times (e.g., five times) longer than the life span of the high-pressure mercury lamp, fewer failures occur due to the life span of the ultraviolet LED elements.

Accordingly, according to the present invention, it is possible, in the configuration for producing the artificial joint component, to achieve a size reduction, to accurately control ultraviolet irradiation intensity, to form a macromolecular layer having a uniform thickness in the artificial joint component, and furthermore to suppress the influence of ultraviolet light on equipment and workers, and to make the occurrence of a malfunction unlikely.

### Effects of the Invention

According to the present invention, it is possible, with regard to a configuration for forming a macromolecular layer on a surface of a base material of an artificial joint component, to achieve a size reduction, to accurately control ultraviolet irradiation intensity, to form a macromolecular layer having a uniform thickness, to suppress the influence of ultraviolet light on equipment and workers, and to make the occurrence of a malfunction unlikely.

### Brief Description of the Drawings

FIG. 1 is a partial cross-sectional view of an artificial hip joint, which includes an artificial joint component formed using a film-producing device according to an embodiment of the present invention.
   FIG. 2 is a perspective view of a film-producing device body and a power supply device of the film-producing device, while omitting an adapter of the film-producing device.
   FIG. 3 is a partial cross-sectional view of the film-producing device body and a base material, showing a state where the film-producing device body is cut along a cross-section extending in the up-down direction.
   FIG. 4 is a bottom view of an LED holder and the like.
   FIG. 5 is a schematic view for illustrating ultraviolet light emitted from each ultraviolet LED element, and shows a state of the ultraviolet LED elements and the like as seen from the side.
   FIG. 6 is a flowchart for illustrating a process of forming a macromolecular layer on the base material.
   FIG. 7 is a diagram for illustrating a process of housing the base material in a work holder, in the process of forming the macromolecular layer on the base material.
   FIG. 8 is a diagram for illustrating a process of installing a lens-shaped jig after injecting a solution, in the process of forming the macromolecular layer on the base material.
   FIG. 9 is a partial cross-sectional view showing a main part of a modification of the present invention.

### Description of Embodiments

Hereinafter, a mode for carrying out the present invention will be described with reference to the drawings. Note that the present invention can be widely applied to a film-producing device and a method for producing an artificial joint component.

### Configuration of artificial joint

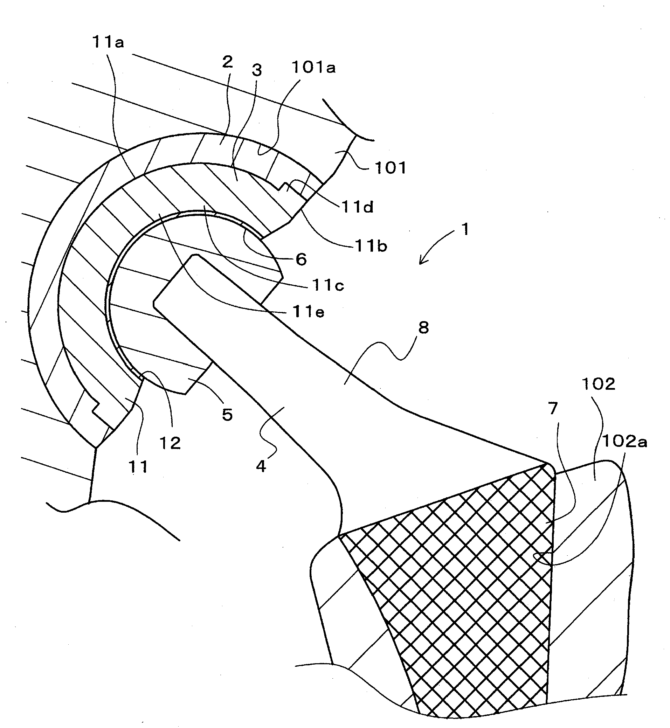

FIG. 1 is a partial cross-sectional view of an artificial hip joint 1, which includes an artificial joint component formed using a film-producing device according to an embodiment of the present invention. Although the present invention is applicable to artificial joints other than an artificial hip joint as described later, the present embodiment will be described using an example of an artificial hip joint.

FIG. 1 shows a state where the artificial hip joint 1 is installed in a patient, as well as showing a part of the pelvis 101 and a part of the femur 102. As shown in FIG. 1, the artificial hip joint 1 is provided as an artificial joint for allowing relative displacement of the femur 102 with respect to the acetabular roof 101a of the pelvis 101.

The artificial hip joint 1 is configured to include a shell 2 and a liner 3 that are arranged on the acetabular roof 101a of the pelvis 100, and a stem 4 and a femoral head ball 5 that are arranged on the femur 102.

The shell 2 and the liner 3 form a spherical joint in cooperation with the femoral head ball 5, and allow movement of the femur 102 with respect to the pelvis 101. The shell 2 is a member formed in a cup shape and having a recess, and is fixed to the acetabular roof 101a of the pelvis 101. The liner 3 is fixed to the shell 2.

The liner 3 is an artificial joint component that is formed in a cup shape and has a recess. A specific configuration of the liner 3 will be described later. A sliding surface 6 is formed on an inner surface at the recessed portion of the liner 3. The femoral head ball 5 is in slidable contact with the sliding surface 6.

The femoral head ball 5 is formed hemispherically. Exemplary materials of the femoral head ball 5 may include metallic materials such as titanium alloy, cobalt-chromium alloy, and stainless steel, macromolecular materials such as polyethylene, and ceramic materials such as alumina and zirconia, which are authorized for use in medical equipment for biological implantation. The femoral head ball 5 is fixed to the stem 4.

The stem 4 is provided as a portion that supports the femoral head ball 5 and is fixed to the femur 102 of the patient. Exemplary materials of the stem 4 may include metallic materials such as titanium, titanium alloy, cobalt-chromium alloy, and stainless steel, which are authorized for use in medical equipment for biological implantation. The stem 4 is formed in an elongated rod shape (stem shape), and has a shape bending at an intermediate portion of the stem 4. The stem 4 is configured to include a stem body 7 and a neck portion 8.

The stem body 7 is inserted into a hole portion formed in the medullary cavity portion 102a of the femur 102, and is fixed to the femur 102. The neck portion 8 extends from the stem body 7. The neck portion 8 extends in an inclining direction with respect to the longitudinal direction of the stem 4. The femoral head ball 5 is fixed to the neck portion 8.

With the above-described configuration, the femoral head ball 5 slides with respect to the sliding surface 6 of the liner 3, and the femur 102 is thereby displaced with respect to the acetabular roof 101a.

Next, the configuration of the liner 3 will be described in more detail. The liner 3 is a substantially hemispherical member that is formed in a cup shape and has a recess. The diameter of the liner 3 is set to about 35 mm to 65 mm, for example.

The liner 3 has a base material 11 and a macromolecular layer 12. At least one of a synthetic resin, a metallic material, and a ceramic material may be given as an exemplary material of the base material 11. Ultra-high-molecular-weight polyethylene (hereinafter referred to also as UHMWPE) may be given as an example of a synthetic resin. Among macromolecular materials, UHMWPE has excellent mechanical properties such as abrasion resistance and deformation resistance, and is suitable for the base material 11. UHMWPE has higher abrasion resistance as its molecular weight is larger. Accordingly, it is preferable to use UHMWPE having a molecular weight of at least 1,000,000 g/mol, and preferably a molecular weight of 3,000,000 g/mol or larger. Alternatively, cross-linked polyethylene prepared by cross-bridging UHMWPE may be used as the base material 11.

As described above, the base material 11 is formed in a cup shape, and has an outer circumferential surface 11a, an end surface 11b, a recess portion 11c, and a flange portion 11d. The outer circumferential surface 11a is provided as a portion that is fixed to an inner circumferential surface of the shell 2. In the present embodiment, the outer circumferential surface 11a is formed in a projecting curved shape. The end surface 11b is formed so as to face in a direction opposite to the outer circumferential surface 11a.

In the present embodiment, the end surface 11b is formed as a flat surface. The recess portion 11c is formed so as to be recessed from the end surface 11b. The recess portion 11c is provided in order to receive the femoral head ball 5 in cooperation with the macromolecular layer 12. The recess portion 11c is recessed inward of the base material 11 from the end surface 11b. An inner surface (predetermined portion) 11e of the recess portion 11c is formed in a recessed curved shape. In the present embodiment, the inner surface 11e is formed in a substantially hemispherical shape. The flange portion 11d is arranged on the opening side of the recess portion 11c.

The flange portion 11d is provided as a portion for fixing the liner 3 to the shell 2. For example, the liner 3 can be fixed to the shell 2 by passing a fixation screw (not shown) through the flange portion 11d and screw-coupling this fixation screw to the shall 2. The macromolecular layer 12 is formed on the inner surface 11e of the recess portion 11c.

The macromolecular layer 12 is provided as a thin film forming the sliding surface 6, and is formed integrally with the base material 11. The thickness of the macromolecular layer 12 is about several nanometers to several hundred micrometers. Note that, in FIG. 1, the macromolecular layer 12 is shown with its thickness exaggerated as compared with its actual thickness. The macromolecular layer 12 is formed over the entire inner surface 11e of the recess portion 11c. The sliding surface 6, which serves as the surface of the macromolecular layer 12, has an uneven shape. In the present embodiment, the sliding surface 6 is formed in a hemispherical shape. A part of the femoral head ball 5 is accommodated by the macromolecular layer 12.

The macromolecular layer 12 is formed by irradiating, with ultraviolet light, a monomer solution having the phosphorylcholine group while this solution is in contact with the inner surface 11e of the recess portion 11c. Next, a film-producing device 10 for forming the macromolecular layer 12 of the liner 3 having the above-described configuration will be described.

### Configuration of film-producing device

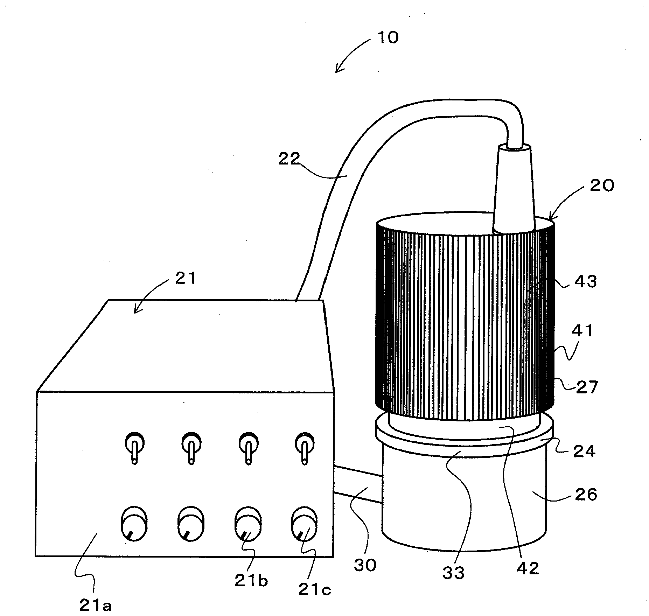

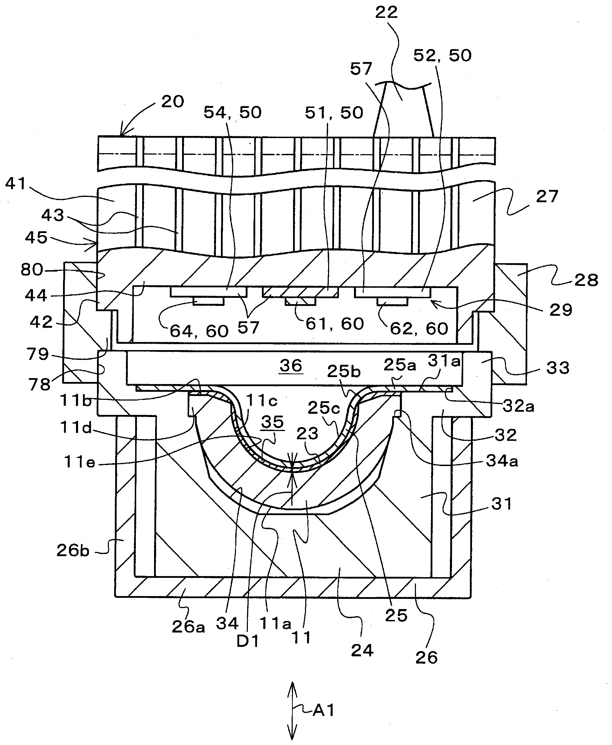

FIG. 2 is a perspective view of a film-producing device body 20 and a power supply device 21 of the film-producing device 10, while omitting a later-described adapter 28 of the film-producing device body 20. FIG. 3 is a partial cross-sectional view of the film-producing device body 20 and the base material 11, showing a state where the film-producing device body 20 is cut along a cross-section extending in the up-down direction (vertical direction). Note that, although the present embodiment will be described based on a state where the film-producing device body 20 is arranged in a vertical orientation, the orientation of the film-producing device body 20 is not particularly limited.

As shown in FIGS. 2 and 3, the film-producing device 10 includes the film-producing device body 20 and the power supply device 21. The film-producing device body 20 is configured such that electric power is supplied thereto from the power supply device 21. The power supply device 21 is a small power supply device whose height, width, and length in the depth direction are about ten centimeters to several tens of centimeters. The power supply device 21 has a casing 21a, a power supply circuit (not shown) accommodated in the casing 21a, and adjustment members 21b and 21c that are connected to the power supply circuit. By the adjustment member 21b being operated by an operator, the brightness of a later-described ultraviolet LED (Light Emitting Diode) element 61 is changed. Furthermore, by the adjustment member 21c being operated by the operator, the brightness of later-described ultraviolet LED elements 62 to 66 is changed. The power supply circuit of the power supply device 21 and the ultraviolet LED elements 61 to 66 of the film-producing device body 20 are electrically connected to each other via a cable 22 or the like.

The film-producing device body 20 irradiates, with ultraviolet light, a solution 23 that is in contact with the base material 11 serving as a work, thereby causing a photograft polymerization reaction, and as a result, forming the macromolecular layer 12 on the base material 11. The film-producing device body 20 is formed in a column shape extending above and below as a whole. In the present embodiment, the film-producing device body 20 is formed in a circular column shape. The film-producing device body 20 is a small device having a diameter of several tens of centimeters and a height of several tens of centimeters. Note that the following description is based on an arrangement of the members at the time when the film-producing device body 20 irradiates the base material 11 with ultraviolet light.

The film-producing device body 20 includes a work holder 24, a lens-shaped jig 25, a heater 26, an LED holder 27, the adapter 28, and a light-emitting device 29.

The work holder 24 is provided as a holding member for holding the base material 11, which serves as a work. The work holder 24 is made of, for example, the same material as the base material 11, or aluminum alloy or the like. If the work holder 24 is made of aluminum alloy, which has excellent heat conductivity, the heat from the heater 26 is quickly transmitted to the solution 23 via the work holder 24 and the base material 11. The work holder 24 has a work holder body 31, a flange portion 32, and a receiving portion 33.

The work holder body 31 is provided as a portion that holds the base material 11. The work holder body 31 is formed in a block shape, and has an upper surface 31a. An accommodating recess portion 34 extending so as to be recessed downward from the upper surface 31a is formed on the work holder body 31. The accommodating recess portion 34 is formed so as to have a size that accommodates the entire base material 11. A loop-shaped step portion 34a is formed in a portion of the accommodating recess portion 34 near the upper surface 31a. This step portion 34a faces upward and can receive the flange portion 11d of the base material 11. The based material 11 in a state of being received by the step portion 34a is arranged at a position that is lower than the position of the upper surface 31a of the work holder body 31. A portion of the accommodating recess portion 34 at a position that is lower than the position of the step portion 34a may be configured to be separate from the outer circumferential surface 11a of the base material 11, or may be configured so as to come into contact with the outer circumferential surface 11a.

The flange portion 32 is formed at the upper-end outer circumferential portion of the work holder body 31 having the above-described configuration. The flange portion 32 is formed in a ring shape, and extends horizontally. A loop-shaped step portion 32a is formed on the upper surface of the flange portion 32. The loop-shaped step portion 32a is provided in order to position the lens-shaped jig 25 with respect to the work holder 24.
The loop-shaped step portion 32a is formed in a cylindrical shape extending in the up-down direction. The diameter of the loop-shaped step portion 32a is set to be substantially the same as the diameter of a later-described flange portion 25a of the lens-shaped jig 25.

The receiving portion 33 is continuous with the outer circumferential portion of the flange portion 32. The receiving portion 33 is provided as a portion that receives the adapter 28. The receiving portion 33 is formed in a cylindrical shape, and extends upward from the flange portion 32. The upper surface of the receiving portion 33 is a horizontal flat surface. The lens-shaped jig 25 is installed in the work holder 24 having the above-described configuration.

The lens-shaped jig 25 is provided in order to form a minute space 35 between this lens-shaped jig 25 and the inner surface 11e of the base material 11 and cause ultraviolet light from the light-emitting device 29 to enter this space 35. The aforementioned space 35 is filled with the solution 23. By thus filling the minute space 35 with the solution 23, the amount of solution 23 to be used at the time of a photograft polymerization reaction is reduced. Note that, although the present embodiment will be described regarding a mode using the lens-shaped jig 25, the lens-shaped jig 25 may be omitted.

The lens-shaped jig 25 is made of a material that can transmit ultraviolet light. A translucent plastic, and an inorganic glass such as silica glass or Pyrex (registered trademark) glass can be given as an example of such a material. The thickness of the lens-shaped jig 25 is set as appropriate so as to have a sufficient strength and such that ultraviolet light can be sufficiently transmitted.

The lens-shaped jig 25 has the flange portion 25a, a first curved portion 25b, and a second curved portion 25c.

The flange portion 25a is provided as a portion received by the upper surface 31a of the work holder body 31. The flange portion 25a is formed in a ring shape, and is in contact with the upper surface 31a of the work holder body 31. The flange portion 25a is surrounded by the loop-shaped step portion 32a of the flange portion 32, thereby being positioned with respect to the work holder 24. In the present embodiment, the lower surface of the flange portion 25a faces the end surface 11b of the base material 11 accommodated by the accommodating recess portion 34. The first curved portion 25b is continuous with the inner circumferential portion of the flange portion 25a.

The first curved portion 25b is provided as a portion that connects the flange portion 25a and the second curved portion 25c to each other. The first curved portion 25b is arranged near the opening of the recess portion 11c of the base material 11. As shown in FIG. 3, the first curved portion 25b is formed in a curved shape that protrudes upward and toward the center axis of the lens-shaped jig 25 in a cross-section extending in the vertical direction. The first curved portion 25b is formed in a ring shape in any cross-section perpendicular to the center axis, and the diameter thereof continuously decreases downward. The second curved portion 25c is continuous with the lower end portion of the first curved portion 25b.

The second curved portion 25c is formed in a curved shape that protrudes downward. In the present embodiment, the second curved portion 25c is formed in a hemispherical shape. The lower portion of the first curved portion 25b and the second curved portion 25c are formed in a shape having a substantially constant interval D1 from any part of the inner surface 11e of the base material 11.

The aforementioned interval D1 is preferably at least 0.1 mm to 20 mm, more preferably 0.2 mm to 5 mm, and still more preferably 0.2 mm to 2 mm. If the interval is 20 mm or smaller, the amount of the solution 23 to be used can be sufficiently reduced. On the other hand, if the lower limit value of the interval D1 is smaller than 0.1 mm, there is a possibility that the lens-shaped jig 25 and the base material 11 will come into contact with each other due to the process tolerance of the members, namely the lens-shaped jig 25, the base material 11, and the work holder 24. If the lower limit value of the interval D1 is 0.1 mm or larger, the macromolecular layer 12 can be favorably formed on the inner surface 11e.

As mentioned above, the space 35 between the base material 11 and the lens-shaped jig 25 is filled with the solution 23. In the present embodiment, the solution 23 is in contact with the inner surface 11e of the base material 11 and the end surface 11b of the base material 11. This solution 23 is a polymerizable (macromolecular) monomer solution having the phosphorylcholine group. This polymerizable monomer is configured to have the phosphorylcholine group at one end and a functional group capable of undergoing graft polymerization with the base material 11 at the other end, and it is thereby possible to form the macromolecular layer 12 on the inner surface 11e of the base material 11 by photograft polymerization.

Examples of the polymerizable monomers contained in the solution 23 include 2-methacryloyloxyethyl phosphorylcholine, 2-acryloyloxyethyl phosphorylcholine, 4-methacryloyloxybutyl phosphorylcholine, 6-methacryloyloxyhexyl phosphorylcholine, ω-methacryloyloxyethylene phosphorylcholine, 4-styryloxybutyl phosphorylcholine, and the like. In particular, 2-methacryloyloxyethyl phosphorylcholine (referred to also as MPC) is preferable.

Each MPC monomer includes the phosphorylcholine group and a polymerizable methacrylate unit. MPC monomers have a feature of capability to easily form high-molecular-weight MPC polymers by means of radical polymerization (Ishihara et al.: Polymer Journal, vol. 22, page 355 (1990)). For this reason, if the macromolecular layer 12 is synthesized from MPC monomers, graft polymerization between the macromolecular layer 12 and the inner surface 11e can be carried out under relatively moderate conditions, and furthermore, a large quantity of phosphorylcholine group can be formed on the inner surface 11e by forming a high-density macromolecular layer.

Note that the macromolecular layer 12 can be formed not only by homopolymers each constituted by a single polymerizable monomer having the phosphorylcholine group, but also by copolymers each constituted by a monomer having the phosphorylcholine group and, for example, another vinyl compound monomer. A function of improving the mechanical strength or the like can thereby be added to the macromolecular layer 12.

The solution 23 that fills the space between the lens-shaped jig 25 having the above-described configuration and the base material 11 is heated by the heater 26. The heater 26 forcibly heats the solution 23 by converting electric energy into heat energy, for example. In the present embodiment, the heater 26 is formed in a cup shape, and has a bottom wall 26a and a circumferential wall 26b.

The work holder body 31 is placed on the bottom wall 26a. The circumferential wall 26b extends upward from the outer circumferential portion of the bottom wall 26a. The circumferential wall 26b is in contact with the flange portion 32. A heat generating member (not shown), such as a lead wire, is buried in the bottom wall 26a and the circumferential wall 26b of the heater 26, and is electrically connected to the power supply device 21 via a cable 30 or the like. The heat generated by the heat generating member is thereby transmitted to the solution 23 via the work holder body 31 and the base material 11, and the temperature of the solution 23 becomes suitable for a photograft polymerization reaction. The light-emitting device 29 that generates the light for this photograft polymerization reaction is held by the LED holder 27.

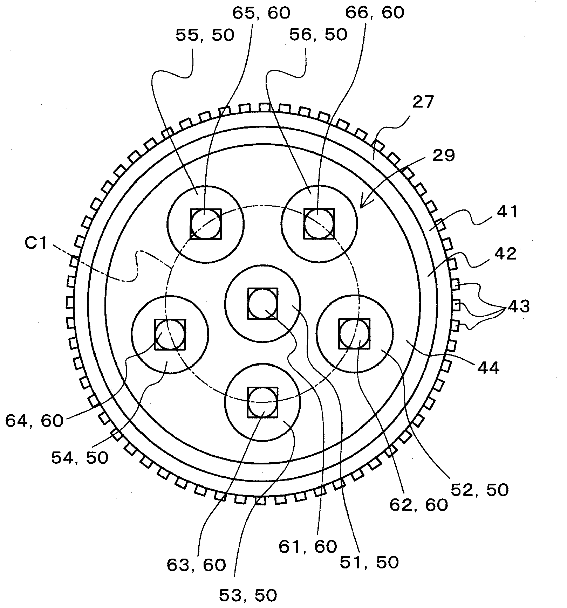

FIG. 4 is a bottom view of the LED holder 27 and the like. As shown in FIGS. 2, 3, and 4, the LED holder 27 is provided as a holding member that holds the light-emitting device 29. The LED holder 27 is formed in a circular column shape, and is arranged above the work holder 24. The LED holder 27 is made of a synthetic resin, an aluminum alloy, or the like. The LED holder 27 and the work holder 24 face each other in a facing direction A1. In the present embodiment, the facing direction A1 is the up-down direction, and is the direction in which ultraviolet LED elements 60 and the work holder 24 face each other. The LED holder 27 has an LED holder body 41 and a receiving portion 42.

The LED holder body 41 has a shape provided with a plurality of fins 43 around an outer shell portion having a circular column shape. The fins 43 are arranged at even intervals in the circumferential direction of the LED holder body 41, and extend vertically. With these fins 43, the heat from the light-emitting device 29 can be efficiently discharged to outside the LED holder 27. In the preset embodiment, the length of the LED holder body 41 is larger than the length of the work holder 24 in the up-down direction. The cable 22 is connected to the upper portion of the LED holder body 41. A cable (not shown) electrically connected to the cable 22 extends within the LED holder body 41. In the present embodiment, the outside diameter of the LED holder body 41 is set to be substantially the same as the outside diameter of the receiving portion 33. A mount portion 44 is formed at a lower end portion of the LED holder body 41. The mount portion 44 is provided in order to support the light-emitting device 29, and is formed in a plate shape that closes the lower end portion of the LED holder body 41. The lower end surface of the mount portion 44 extends horizontally. The receiving portion 42 extends from the lower end portion of the LED holder body 41.

The receiving portion 42 is provided as a portion received by the upper surface of a loop-shaped projecting portion 79 of the adapter 28. The receiving portion 42 is formed in a cylindrical shape. A cylindrical portion extends from the inner circumferential portion of the lower surface of the receiving portion 42 toward the work holder 24. The outer circumferential portion of the lower surface of the receiving portion 42 is received by the upper surface of the loop-shaped projecting portion 79. The receiving portion 33 is provided as a portion received by the lower surface of the loop-shaped projecting portion 79 of the adapter 28. By the receiving portion 42 and the receiving portion 33 being received by the loop-shaped projecting portion 79, the LED holder 27 and the work holder 24 are arranged at desired positions.

With the above-described configuration, a light emission space 36 is formed between the LED holder 27 and the work holder 24. The light emission space 36 is a space that can be reached by light from the light-emitting device 29, and includes the space between the later-described ultraviolet LED elements 60 and the work holder 24. The light emission space 36 is a space demarcated by the mount portion 44 of the LED holder body 41, the adapter 28, the flange portion 32, and the work holder body 31.

The aforementioned light emission space 36 is optically shielded from the outside space by a light shielding member 45 including the LED holder 27 and the work holder 24. Being "optically shielded" in this case means that the light emission space 36 is shielded from light, regardless of whether a fluid such as water is allowed to pass through or not. The outside space refers to a space other than the light emission space 36. The space 35 exists within the light emission space 36.

In the present embodiment, the light shielding member 45 is a complex member formed by the mount portion 44 of the LED holder body 41, the adapter 28, the flange portion 32, and the work holder body 31. That is to say, the light shielding member 45 is formed by the members for forming the light emission space 36. With the provision of the light shielding member 45, the solution 23 can be prevented from being irradiated with light other than the light from the light-emitting device 29.

The light-emitting device 29 emits ultraviolet light to the light emission space 36, in particular the space 35, thereby irradiating the solution 23 with the ultraviolet light. A photograft polymerization reaction is thereby caused around the inner surface 11e of the base material 11, and as a result, the macromolecular layer 12 is formed on the inner surface 11e.

The light-emitting device 29 includes a plurality of LED units 50 (51, 52, 53, 54, 55, 56) that are collectively held by the LED holder 27. In the present embodiment, six LED units 50 (51, 52, 53, 54, 55, 56) are provided. Note that, in the following description, the LED units 51 to 56 are also referred to collectively as the LED units 50. The LED units 50 are fixed to the mount portion 44 of the LED holder 27. The LED unit 51 is arranged so as to face the bottom portion of the recess portion 11c of the base material 11 in the facing direction A1. The LED units 52 to 56 are arranged so as to surround the LED unit 51. More specifically, the LED units 52 to 56 are arranged on a virtual reference circle C1 that is concentric with the LED holder body 41, at even intervals in the circumferential direction of the reference circle C1.

The LED units 50 each have a base plate 57. A corresponding ultraviolet LED element 60(61, 62, 63, 64, 65, 66) is installed on each base plate 57. Note that the ultraviolet LED elements 61 to 66 are also referred to collectively as the ultraviolet LED elements 60. The ultraviolet LED elements 60 are separately arranged. Each base plate 57 is a rigid base plate, for example, and has a thickness of about several millimeters. A cable (not shown) electrically connected to the cable 22 is connected to each base plate 57. Electric power from the power supply device 21 is thereby supplied to the ultraviolet LED elements 60 via the cable 22, the base plates 57, and the like.

In the present embodiment, the base plates 57 are configured to be attachable to and detachable from the mount portion 44, and with this configuration, the positions of the ultraviolet LED elements 60 with respect to the base material 11 can be changed. The method for attaching the base plates 57 to the mount portion 44 is not particularly limited, and for example, the base plates 57 are attached thereto using screw members (not shown).

Each ultraviolet LED element 60 is installed at the center of the corresponding base plate 57. The ultraviolet LED elements 60 receive the electric power supplied from the power supply device 21, and thereby emit ultraviolet light. The wavelength of the light emitted by each ultraviolet LED element 60 is about 300 nm to 400 nm, for example, and preferably is 315 nm to 400 nm. If the aforementioned wavelength is 315 nm or larger, emission of ultraviolet-B to the light emission space 36 from each ultraviolet LED element 60 can be suppressed.

The width of the wavelength of the light emitted by each ultraviolet LED element 60 is about several nanometers, and the wavelength width is set to be sufficiently narrow. Accordingly, it can be said that the light emitted from the ultraviolet LED elements 60 is substantially only ultraviolet light. The length of a single side of each ultraviolet LED element 60 is about several millimeters. On the other hand, as mentioned above, the diameter of the base material 11 is about 35 mm to 65 mm, and the size of each ultraviolet LED element 60 is sufficiently smaller than the size of the base material 11.

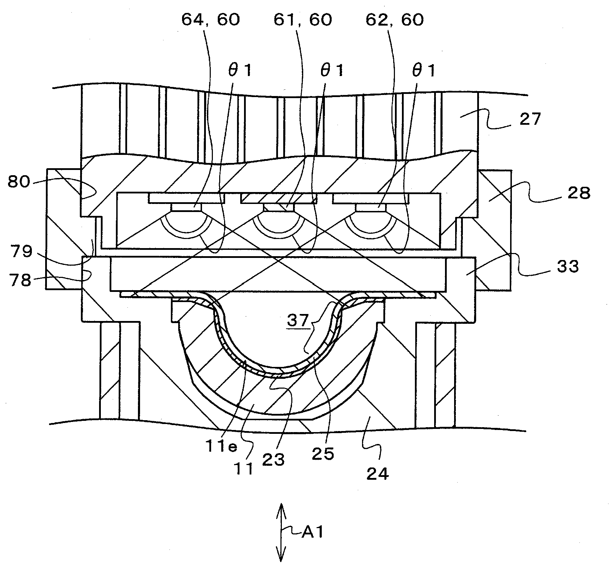

FIG. 5 is a schematic view for illustrating the ultraviolet light emitted from each ultraviolet LED element 60, and shows a state of the ultraviolet LED elements 60 and the like as seen from the side. FIG. 5 shows the ultraviolet LED elements 61, 62, and 64 among the ultraviolet LED elements 61 to 66. As shown in FIGS. 4 and 5, the irradiation angle θ1 of the ultraviolet light emitted from each ultraviolet LED element 60 is about 30° to 150°, for example. In the present embodiment, the irradiation angle θ1 is set to about 115°. The ultraviolet LED elements 60 have a high directivity of the emitted ultraviolet light, and it is easy to irradiate desired positions with the light from the ultraviolet LED elements 60. Moreover, the ultraviolet light emitted from each ultraviolet LED element 60 to the inner surface 11e has a substantially uniform illuminance on the inner surface 11e.

In the present embodiment, the ultraviolet LED element 61 faces the bottom portion of the inner surface 11e in the facing direction A1, and irradiates the entire inner surface 11e with ultraviolet light. The ultraviolet LED element 62 irradiates, with ultraviolet light, an area of the inner surface 11e that directly faces the ultraviolet LED element 62. On the other hand, the ultraviolet LED element 62 cannot directly irradiate, with ultraviolet light, a portion (e.g., area 37) of the curved inner surface 11e that does not directly face the ultraviolet LED element 62. Similarly, each of the ultraviolet LED elements 63 to 66 irradiates, with ultraviolet light, an area of the curved inner surface 11e that directly faces the corresponding ultraviolet LED element, but cannot directly irradiate, with ultraviolet light, a portion of the inner surface 11e that does not directly face the corresponding ultraviolet LED element. However, as mentioned above, the ultraviolet LED elements 62 to 66 are arranged at even intervals in the circumferential direction of the reference circle C1. The entire inner surface 11e excluding the bottom portion thereof is thereby directly irradiated with ultraviolet light from the plurality of ultraviolet LED elements 60. In this manner, the ultraviolet LED elements 60 irradiate the solution 23 with the ultraviolet light by irradiating, with the ultraviolet light, an area wider than the area that a single ultraviolet LED element 60 individually irradiates.

In the present embodiment, an adjustment mechanism for adjusting the illuminance of the ultraviolet light in the solution 23 and the inner surface 11e is provided. Specifically, the ultraviolet LED elements 60 are configured to output ultraviolet light having a brightness corresponding to the electric power supplied by the power supply device 21, which serves as the adjustment mechanism. More specifically, an exemplary configuration for changing the brightness may be a configuration in which the value of the current supplied to the ultraviolet LED elements 60 is changed, or in which different duty ratios of the current supplied to the respective ultraviolet LED elements 60 are set by means of PWM (Pulse Width Modulation) control, or the like. In the present embodiment, the power supply device 21 changes the current supplied to the ultraviolet LED elements 60 in accordance with the adjustment members 21b and 21c (see FIG. 2) of the power supply device 21 being operated. For the aforementioned reason, in the present embodiment, it is hard for the light from the ultraviolet LED elements 62 to 66 to reach the bottom portion of the inner surface 11e.

Accordingly, in the present embodiment, a sufficient amount of ultraviolet light needs to be supplied to the bottom portion of the inner surface 11e by the ultraviolet LED element 61. For this reason, in the present embodiment, the current supplied to the ultraviolet LED element 61 is set to be larger than the current supplied to the ultraviolet LED element 62 (63 to 66). The brightness of the ultraviolet light of the ultraviolet LED element 61 is thereby made larger than the brightness of the ultraviolet light of each of the ultraviolet LED elements 62 to 66. As a result, the bottom portion of the inner surface 11e is irradiated with ultraviolet light at the illuminance similar to that in a portion of the inner surface 11e other than the bottom portion.

As shown in FIGS. 2, 3, and 4, the relative position of the light-emitting device 29 with respect to the base material 11 is accurately set by the adapter 28. In the present embodiment, the adapter 28 positions the work holder 24 and the LED holder 27 such that the work holder 24 and the LED holder 27 are coaxially arranged. The loop-shaped projecting portion 79 of the adapter 28 performs positioning such that the distance between the work holder 24 and the LED holder 27 is a desired value.

The adapter 28 is formed in a cylindrical shape. The inner portion of the adapter 28 has a first fitting portion 78, the loop-shaped projecting portion 79, and a second fitting portion 80. The first fitting portion 78, the loop-shaped projecting portion 79 and the second fitting portion 80 are arranged in this order in the up-down direction.

The first fitting portion 78 is a cylindrical surface provided as a portion that is fitted to the receiving portion 33 of the work holder 24. When the adapter 28 is in a separated state, the diameter of the first fitting portion 78 is substantially the same as the diameter of the outer circumferential surface of the receiving portion 33. The second fitting portion 80 is arranged above the first fitting portion 78.

The second fitting portion 80 is a cylindrical surface provided as a portion that is fitted to the lower end portion of the LED holder body 41. When the adapter 28 is in a separated state, the diameter of the second fitting portion 80 is substantially the same as the outside diameter of the lower end portion of the LED holder body 41. The second fitting portion 80 is arranged coaxially with the first fitting portion 78. The loop-shaped projecting portion 79 is arranged below the second fitting portion 80.

The loop-shaped projecting portion 79 is formed in a ring shape, and is received by the upper end surface of the receiving portion 33. The lower surface of the loop-shaped projecting portion 79 extends horizontally, and is received by the upper surface of the receiving portion 33 of the work holder 24. The upper surface of the loop-shaped projecting portion 79 is provided as a portion that is received by the receiving portion 42. The loop-shaped projecting portion 79 is arranged in a state of separating the receiving portion 33 and the receiving portion 42 from each other in the facing direction A1. The schematic configuration of the film-producing device 10 is as described above.

### Process of forming macromolecular layer on inner surface of base material

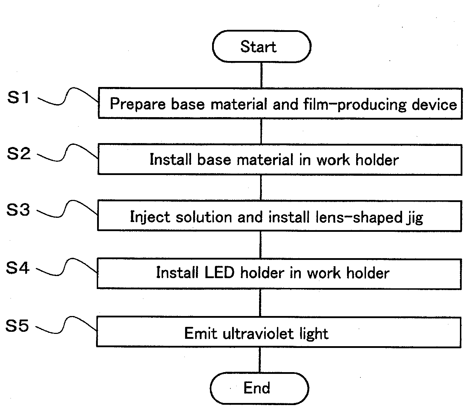

Next, a process of forming the macromolecular layer 12 on the base material 11 using the film-producing device 10 will be described with reference to FIG. 6 and the like. FIG. 6 is a flowchart for illustrating the process of forming the macromolecular layer 12 on the base material 11. When forming the macromolecular layer 12 on the base material 11, initially, the base material 11 and the film-producing device 10 are prepared (step S1).

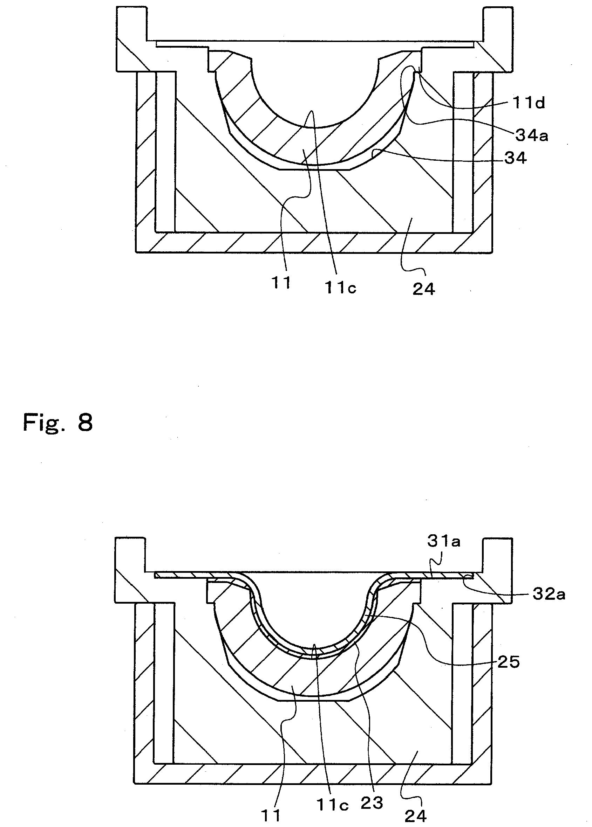

Next, as shown in FIGS. 6 and 7, the base material 11 is installed in the work holder 24 in a state where the LED holder 27 and the adapter 28 are detached therefrom (step S2). At this time, the flange portion 11d of the base material 11 is placed on the step portion 34a of the work holder 24. The base material 11 is thereby arranged within the accommodating recess portion 34 of the work holder 24. Next, as shown in FIGS. 6 and 8, a predetermined amount (e.g., several cubic centimeters) of the solution 23 is supplied to the recess portion 11c of the base material 11, and the lens-shaped jig 25 is thereafter placed on the upper surface 31a of the work holder body 31 and is surrounded by the step portion 32a (step S3). Positioning of the base material 11 with respect to the work holder 24 and positioning of the lens-shaped jig 25 with respect to the work holder 24 are thereby completed. Furthermore, a state in which the solution 23 is in contact with the inner surface 11e is achieved.

Next, as shown in FIGS. 6 and 3, the LED holder 27 is installed in the work holder 24 (step S4). Specifically, the first fitting portion 78 of the adapter 28 is fitted to the outer circumferential portion of the receiving portion 33 of the work holder 24, and the second fitting portion 80 of the adapter 28 is fitted to the lower end portion of the LED holder body 41. The loop-shaped projecting portion 79 of the adapter 28 receives the receiving portion 33, and also receives the receiving portion 42. Positioning of the light-emitting device 29 and the base material 11 is thereby completed.

Next, as shown in FIGS. 5 and 6, ultraviolet light is directly emitted to the solution 23 and the inner surface 11e of the base material 11 (step S5). Specifically, the ultraviolet LED elements 60 emit ultraviolet light, using the electric power supplied from the power supply device 21. A graft polymerization reaction is thereby caused in the monomers contained in the solution 23. As a result, the macromolecular layer 12 is completed on the inner surface 11e of the base material 11.

Note that the base material 11 in which the macromolecular layer 12 is formed can be removed from the work holder 24 after the LED holder 27 and the lens-shaped jig 25 are removed from the work holder 24.

With the above-described film-producing device 10, ultraviolet light is emitted by the plurality of ultraviolet LED elements 60. Each ultraviolet LED element 60 is formed to have a shape that is sufficiently small relative to the base material 11 of the liner 3. Accordingly, a high-pressure mercury lamp or the like having a shape that is significantly larger than the shape of the base material 11 of the liner 3 does not need to be used to produce the liner 3. Since the shape of each ultraviolet LED element 60 is small, even if a plurality of ultraviolet LED elements 60 are arranged, the entire space occupied by the ultraviolet LED elements 60 is small. Moreover, the amount of the heat generated by the ultraviolet LED elements 60 is significantly smaller than that of a high-pressure mercury lamp, and it is not necessary to prepare a large-scale cooling device such as a chiller. As a result, a reduction in the size of the film-producing device 10 can be achieved through a reduction in the size of the configuration for emitting ultraviolet.

With the ultraviolet LED elements 60, the brightness of the ultraviolet LED elements 60 can be easily finely adjusted by adjusting the current supplied to the ultraviolet LED elements 60 or the like. For this reason, the illuminance of the ultraviolet light in the solution 23 can be easily accurately adjusted. As a result, the macromolecular layer 12 having a uniform thickness can be easily formed.

The ultraviolet LED elements 60 have a higher directivity of the emitted light than that of a high-pressure mercury lamp, and can emit ultraviolet light at a more uniform intensity. Moreover, the ultraviolet LED elements 60 are arranged separately from each other. As a result, ultraviolet light of uniform intensity can be emitted over a wide area. The progress of photograft polymerization on the inner surface 11e of the base material 11 can thereby be made more uniform. Accordingly, the macromolecular layer 12 having a uniform thickness can be formed on the inner surface 11e of the base material 11.

The wavelength width of the ultraviolet light emitted by the ultraviolet LED elements 60 is significantly narrower than the wavelength width of the ultraviolet light emitted by the high-pressure mercury lamp. For this reason, the ultraviolet LED elements 60 can be configured not to emit ultraviolet-B, which is considered unnecessary for photograft polymerization. As a result, it is possible to suppress deterioration of equipment due to ultraviolet-B, in the members of the film-producing device 10 that are irradiated with the light from the ultraviolet LED elements 60, such as the work holder 24. Moreover, it is possible to suppress the exposure of workers who produce the liner 3 to ultraviolet-B, which is also preferable in terms of the health management of workers.

Each ultraviolet LED element 60 is installed on the strong base plate 57, and is easier to handle than the high-pressure mercury lamp, which has a large fragile glass portion. Moreover, as mentioned above, a glass portion such as a chiller for cooling the ultraviolet LED elements 60 is not necessary. As a result, with the ultraviolet LED elements 60, it is possible to make the occurrence of a malfunction due to breakage unlikely. Moreover, by using the ultraviolet LED elements 60 whose life span is several times (e.g., five times) longer than the life span of the high-pressure mercury lamp, fewer failures occur due to the life span of the ultraviolet LED elements 60.

Accordingly, with the film-producing device 10, it is possible to achieve a size reduction, to accurately control the ultraviolet irradiation intensity, to form the macromolecular layer 12 having a uniform thickness, to suppress the influence of ultraviolet light on equipment and workers, and to make the occurrence of a malfunction unlikely.

With the film-producing device 10, the plurality of ultraviolet LED elements 60 can be collectively operated using the LED holder 27. The ultraviolet LED elements 60 can thereby be collectively displaced with respect to the base material 11 and the work holder 24. Accordingly, it can be made easier for a worker to handle the ultraviolet LED elements 60.

The film-producing device 10 is provided with the adjustment mechanism for adjusting the illuminance of ultraviolet light in the solution 23 and the inner surface 11e of the base material 11. By thus enabling the illuminance of the ultraviolet light in the solution 23 and the inner surface 11e to be adjusted, the uniformity of the thickness of the macromolecular layer 12 formed by means of photograft polymerization can be further increased. In particular, the inner surface 11e of the base material 11 is uneven. Accordingly, adjustment of the illuminance of the ultraviolet LED element 61 in accordance with the shape of the inner surface 11e is favorable for further increasing the uniformity of the thickness of the macromolecular layer 12 formed on the inner surface 11e.

In particular, in the present embodiment, the current supplied to the ultraviolet LED element 61 is configured to be different from the current supplied to each of the ultraviolet LED elements 62 to 66. Thus, the ultraviolet LED elements 60 are configured using LEDs whose brightness can be easily adjusted by means of electric power adjustment. The illuminance of the ultraviolet light in the solution 23 and the inner surface 11e of the base material 11 can thereby be easily adjusted. As a result, the illuminance of the ultraviolet light in the solution 23 and the inner surface 11e of the base material 11 can be easily adjusted. The illuminance of the ultraviolet light in the solution 23 and the inner surface 11e of the base material 11 can thereby be made more uniform.

Since the film-producing device 10 is provided with the light shielding member 45, light other than the light from the ultraviolet LED elements 60 can be prevented from entering the light emission space 36 between the ultraviolet LED elements 60 and the work holder 24. A non-uniform graft polymerization reaction in the solution 23 and the inner surface 11e due to unnecessary ultraviolet light from the outside can thereby be prevented. As a result, the thickness of the macromolecular layer 12 can be made more uniform.

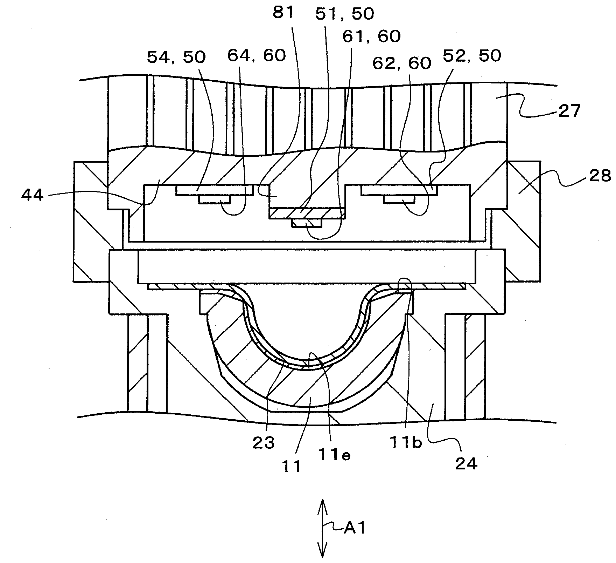

Although an embodiment of the present invention has been described above, the present invention is not limited to the above embodiment, and various modifications are possible within the scope recited in the claims.
(1) The above embodiment has been described, taking an example of a mode in which, in order to adjust the illuminance of the ultraviolet light on the inner surface 11e, the electric power supplied to the ultraviolet LED element 61, which is one of the ultraviolet LED elements 60, is set to be larger than the electric power supplied to each of the other ultraviolet LED elements 62 to 66. However, this need not be the case. For example, as shown in FIG. 9, an adjustment mechanism for adjusting the illuminance may be formed by arranging the ultraviolet LED elements 60 in at least two positions in the facing direction A1.
   Note that the following description is mainly about the difference from the embodiment shown in FIGS. 1 to 8, and the same configuration will be given the same reference numerals in the drawings and will not be described. As shown in FIG. 9, the ultraviolet LED element 61 is fixed to an extension portion 81 that extends from the mount portion 44 of the LED holder 27. The extension portion 81 is a block-shaped portion extending downward from the mount portion 44 of the LED holder body 41. The LED unit 51 including the ultraviolet LED element 61 is fixed to the lower surface of the extension portion 81. The length of the extension portion 81 from the mount portion 44 is set as appropriate in accordance with the distance from the end surface 11b of the base material 11 to the bottom portion of the inner surface 11e, or the like. With the above configuration, the mount portion 44 and the extension portion 81 constitute the adjustment mechanism.
   Thus, the ultraviolet LED elements 60 are arranged in at least two positions in the facing direction A1. That is to say, with a simple configuration in which the positions of the ultraviolet LED elements 61 to 66 are different in the facing direction A1, the illuminance of the ultraviolet light in the solution 23 and the inner surface 11e can be made more uniform. In particular, with the aforementioned different positions of the ultraviolet LED elements 60 with respect to the inner surface 11e that is uneven and on which a shadow is formed by the light from some of the ultraviolet LED elements 60, the illuminance of the ultraviolet light on the inner surface 11e can be made more uniform.
(2) Although the above embodiment has been described, taking an example of a mode in which the macromolecular layer is formed on the inner surface having a recessed curved shape, this need not be the case. The film-producing device in the present embodiment can form the macromolecular layer on a base material in which the predetermined portion is a flat surface, and can form the macromolecular layer on a base material in which the predetermined portion has a shape other than a flat shape. The shape of the predetermined portion may be a simple shape, or may be a complicated uneven shape. In this case, with a simple configuration in which at least one of the changing of the position of each ultraviolet LED element with respect to the LED holder and the changing of the illuminance of each ultraviolet LED element, in accordance with the shape of the predetermined portion, is applied, a macromolecular layer having a uniform thickness can be formed in a plurality of base materials with the predetermined portion having different shapes.
(3) Although the above embodiment has been described, taking an example of a mode in which a configuration for adjusting the intensity of the ultraviolet light on the inner surface 11e of the base material 11 is provided, this need not be the case. For example, the LED units 50 (51, 52, 53, 54, 55, 56) may be arranged on the mount portion 44, and the current supplied to each ultraviolet LED element 60 may be the same.
(4) Although the above embodiment has been described, taking an example of the liner of the artificial hip joint as the artificial joint component in which the macromolecular layer is formed by the film-producing device, this need not be the case. For example, a component having a sliding surface in an artificial shoulder joint, an artificial knee joint, an artificial elbow joint, an artificial ankle joint, an artificial finger joint, or the like can be given as the above-described artificial joint component.

### Industrial Applicability

The present invention can be widely applied to a film-producing device used in production of an artificial joint component, and a method for producing an artificial joint component.

### Descriptions of Reference Numerals

- 3: Liner (artificial j oint component)
- 6: Sliding surface
- 10: Film-producing device
- 11: Base material (work)
- 11e: Inner surface (predetermined portion in which macromolecular layer is formed)
- 12: Macromolecular layer
- 23: Solution
- 24: Work holder
- 60: Ultraviolet LED element

## Claims

1. A film-producing device (10), the device (10) comprising:
an LED holder (27) that collectively holds a plurality of ultraviolet LED elements (60) configured to emit ultraviolet light; and
a work holder (24) with a work holder body (31), wherein an accommodating recess portion (34) is formed on the work holder body (31) so as to have a size that accommodates an entire base material (11) for holding the base material (11) in a state where a solution (23) containing a macromolecular monomer is brought into contact with a sliding surface (6) of the base material (11) having an uneven shape
wherein the plurality of ultraviolet LED elements (60) are arranged separately from each other, and irradiate the solution (23) on the sliding surface (6) with the ultraviolet light by irradiating, with the ultraviolet light, a wider area of the sliding surface (6) than an area that each of the ultraviolet LED elements (60) individually irradiates so that a macromolecular layer (12) is formed by photograft polymerization on the sliding surface (6),
**characterized in that**
the base material (11) is of an artificial joint composite (1);
the work holder body (31) supports the LED holder (27) via an adapter (28), and
a heater (26) for heating the solution (23) is attached to the work holder body (31), wherein heat generated by the heater (26) is transmitted to the solution (23) via the work holder body (31).

2. The film-producing device (10) according to claim 1,
wherein the film-producing device (10) is provided with an adjustment mechanism (21) for adjusting illuminance of the ultraviolet light in the solution (23) by differentiating an amount of electric power supplied to some of the ultraviolet LED elements (60) from an amount of electric power supplied to the other of the ultraviolet LED elements (60).

3. The film-producing device (10) according to any one of claim1 or 2,
further comprising a light shielding member (45) that optically shields a space (36) between the plurality of ultraviolet LED elements (60) and the work holder (24) from an outside space.

4. A method for producing an artificial joint composite (1), the method comprising:
a solution supply step of bringing a solution (23) containing a macromolecular monomer into contact with an uneven shaped sliding surface (6) of a base material (11) of the artificial joint composite (1), and
an irradiation step of irradiating the solution (23) on the sliding surface (6) with ultraviolet light by a plurality of ultraviolet LED elements (60), wherein a macromolecular layer (12) is formed by photograft polymerization on the sliding surface (6), and the ultraviolet LED elements (60) are arranged separately from each other thus irradiating, with the ultraviolet light, a wider area on the sliding surface (6) than an area that each of the ultraviolet LED elements (60) individually irradiates,
wherein an illuminance of the ultraviolet light in the solution (23) is adjusted by arranging the plurality of ultraviolet LED elements in a direction (A1) in which the plurality of ultraviolet LED elements (60) and the sliding surface (6) face each other; and
the solution (23) is heated using a heater (26).

## Patentansprüche

1. Filmherstellungsvorrichtung (10), wobei die Vorrichtung (10) Folgendes umfasst:
einen LED-Halter (27), der gemeinsam mehrere Ultraviolett-LED-Elemente (60) hält, die dafür ausgestaltet sind, ultraviolettes Licht auszusenden; und
einen Arbeitshalter (24) mit einem Arbeitshalterkörper (31), wobei ein Aufnahmeaussparungsabschnitt (34) so in dem Arbeitshalterkörper (31) ausgebildet ist, dass seine Größe dafür eingerichtet ist, ein gesamtes Basismaterial (11) aufzunehmen, um das Basismaterial (11) in einem Zustand zu halten, in dem eine Lösung (23), die ein makromolekulares Monomer enthält, in Kontakt mit einer Gleitfläche (6) des Basismaterials (11) gebracht wird, die eine unebene Form aufweist,
wobei die mehreren Ultraviolett-LED-Elemente (60) separat voneinander angeordnet sind und die Lösung (23) auf der Gleitfläche (6) mit dem ultravioletten Licht bestrahlen, indem mit dem ultravioletten Licht ein breiterer Bereich der Gleitfläche (6) bestrahlt wird als ein Bereich, den jedes der Ultraviolett-LED-Elemente (60) einzeln bestrahlt, so dass eine makromolekulare Schicht (12) durch Photopfropfpolymerisation auf der Gleitfläche (6) entsteht,
**dadurch gekennzeichnet, dass**
das Basismaterial (11) aus einem Verbundmaterial (1) für ein künstliches Gelenk besteht;
der Arbeitshalterkörper (31) den LED-Halter (27) über einen Adapter (28) stützt, und
eine Heizvorrichtung (26) zum Erwärmen der Lösung (23) an dem Arbeitshalterkörper (31) angebracht ist, wobei durch die Heizvorrichtung (26) erzeugte Wärme über den Arbeitshalterkörper (31) zu der Lösung (23) übertragen wird.

2. Filmherstellungsvorrichtung (10) nach Anspruch 1, wobei die Filmherstellungsvorrichtung (10) mit einem Justiermechanismus (21) versehen ist, um die Beleuchtungsstärke des ultravioletten Lichts in der Lösung (23) einzustellen, indem ein Betrag an elektrischem Strom, der einigen der Ultraviolett-LED-Elemente (60) zugeführt wird, von einem Betrag an elektrischem Strom, der den anderen der Ultraviolett-LED-Elemente (60) zugeführt wird, differenziert wird.

3. Filmherstellungsvorrichtung (10) nach einem der Ansprüche 1 und 2, die des Weiteren ein Lichtabschirmelement (45) umfasst, das einen Raum (36) zwischen den mehreren Ultraviolett-LED-Elementen (60) und dem Arbeitshalter (24) von einem äußeren Raum optisch abschirmt.

4. Verfahren zum Herstellen eines Verbundmaterials (1) für ein künstliches Gelenk, wobei das Verfahren Folgendes umfasst:
einen Lösungszuführschritt, bei dem eine Lösung (23), die ein makromolekulares Monomer enthält, in Kontakt mit einer uneben geformten Gleitfläche (6) eines Basismaterials (11) des Verbundmaterials (1) für ein künstliches Gelenk gebracht wird, und
einen Bestrahlungsschritt zum Bestrahlen der Lösung (23) auf der Gleitfläche (6) mit ultravioletten Licht durch mehrere Ultraviolett-LED-Elemente (60), wobei eine makromolekulare Schicht (12) durch Photopfropfpolymerisation auf der Gleitfläche (6) gebildet wird, und die Ultraviolett-LED-Elemente (60) separat voneinander angeordnet sind, so dass mit dem ultravioletten Licht ein breiterer Bereich der Gleitfläche (6) bestrahlt wird als ein Bereich, den jedes der Ultraviolett-LED-Elemente (60) einzeln bestrahlt,
wobei eine Beleuchtungsstärke des ultravioletten Lichts in der Lösung (23) eingestellt wird, indem die mehreren Ultraviolett-LED-Elemente in einer Richtung (A1) angeordnet werden, in der die mehreren Ultraviolett-LED-Elemente (60) und die Gleitfläche (6) einander zugewandt sind; und
die Lösung (23) unter Verwendung einer Heizvorrichtung (26) erwärmt wird.

## Revendications

1. Dispositif de production de film (10), le dispositif (10) comprenant
un support de LED (27) qui contient collectivement une pluralité d'éléments à LED ultraviolets (60) configurés pour émettre une lumière ultraviolette ; et
un support de travail (24) avec un corps de support de travail (31), dans lequel une partie d'évidement de logement (34) est formée sur le corps de support de travail (31) de manière à avoir une taille permettant de recevoir un matériau de base entier (11) pour maintenir le matériau de base (11) dans un état où une solution (23) contenant un monomère macromoléculaire est mise en contact avec une surface de glissement (6) du matériau de base (11) ayant une forme irrégulière,
dans lequel la pluralité d'éléments à LED ultraviolets (60) sont agencés séparément les uns des autres et irradient la solution (23) sur la surface de glissement (6) avec la lumière ultraviolette en irradiant, avec la lumière ultraviolette, une zone plus large de la surface de glissement (6) qu'une zone que chacun des éléments à LED ultraviolets (60) irradie individuellement, de sorte qu'une couche macromoléculaire (12) soit formée par polymérisation par photogreffe sur la surface de glissement (6),
**caractérisé en ce que**
le matériau de base (11) est constitué d'un composite d'articulation artificielle (1) ;
le corps de support de travail (31) supporte le support de LED (27) via un adaptateur (28), et
un dispositif de chauffage (26) pour chauffer la solution (23) est fixé au corps de support de travail (31), dans lequel la chaleur générée par le dispositif de chauffage (26) est transmise à la solution (23) via le corps de support de travail (31).

2. Dispositif de production de film (10) selon la revendication 1,
dans lequel le dispositif de production de film (10) est pourvu d'un mécanisme de réglage (21) pour régler l'éclairement de la lumière ultraviolette dans la solution (23) en différenciant une quantité d'énergie électrique fournie à certains des éléments à LED ultraviolets (60) d'une quantité d'énergie électrique fournie aux autres des éléments à LED ultraviolets (60).

3. Dispositif de production de film (10) selon l'une quelconque des revendications 1 ou 2,
comprenant en outre un élément de protection contre la lumière (45) qui protège optiquement un espace (36) entre la pluralité d'éléments à LED ultraviolets (60) et le support de travail (24) d'un espace extérieur.

4. Procédé de fabrication d'un composite d'articulation artificielle (1), le procédé comprenant :
une étape de fourniture de solution consistant à amener une solution (23) contenant un monomère macromoléculaire en contact avec une surface de glissement de forme irrégulière (6) d'un matériau de base (11) du composite d'articulation artificielle (1), et
une étape d'irradiation consistant à irradier la solution (23) sur la surface de glissement (6) avec une lumière ultraviolette par une pluralité d'éléments à LED ultraviolets (60), dans lequel une couche macromoléculaire (12) est formée par polymérisation par photogreffe sur la surface de glissement (6), et les éléments à LED ultraviolets (60) sont agencés séparément les uns des autres, irradiant ainsi, avec la lumière ultraviolette, une zone plus large sur la surface de glissement (6) qu'une zone que chacun des éléments à LED ultraviolets (60) irradie individuellement,
dans lequel un éclairement de la lumière ultraviolette dans la solution (23) est réglé en agençant la pluralité d'éléments à LED ultraviolets dans une direction (A1) dans laquelle la pluralité d'éléments à LED ultraviolets (60) et la surface de glissement (6) se font mutuellement face ; et
la solution (23) est chauffée à l'aide d'un dispositif de chauffage (26).
